# EUROPÄISCHE PATENTSCHRIFT

(11) **EP 2 793 678 B1**
(45) Veröffentlichungstag und Bekanntmachung des Hinweises auf die Patenterteilung: **06.03.2019**
(21) Anmeldenummer: 12824896.0
(22) Anmeldetag: 21.12.2012
(51) Int. Cl.: A61B 1/05, H02G 1/12, H04N 5/225

(54) **OPTISCHE SENSORANORDNUNG UND VERFAHREN ZUR HERSTELLUNG DER SELBEN**
OPTICAL SENSOR ARRANGEMENT AND METHOD FOR PRODUCING SAME
ENSEMBLE DE DÉTECTION OPTIQUE ET PROCÉDÉ DE FABRICATION DUDIT SYSTÈME

(30) Priorität: 23.12.2011 CH 20462011
(43) Veröffentlichungstag der Anmeldung: 29.10.2014
(73) Patentinhaber: AWAIBA Consultadoria, Desenvolvimento e Comércio de Componentes Microelectrónicos, Unipessoal, Lda., Madeira (PT)
(72) Erfinder: WÄNY, Martin, CH-1400 Yverdon-les-Bains (CH)
(74) Vertreter: Epping - Hermann - Fischer
(86) Internationale Anmeldenummer: PCT/CH2012/000279
(87) Internationale Veröffentlichungsnummer: WO 2013/091123

(56) Entgegenhaltungen:
- EP-A1- 1 104 182
- DE-A1- 10 017 036
- DE-B3- 10 332 845
- JP-A- H0 784 193
- US-A1- 2005 285 973
- US-A1- 2008 091 064

## Beschreibung

### Technischer Bereich

Die Erfindung betrifft einen Endoskop mit einer optischen Sensoranordnung mit einer distalen Seite und einer proximalen Seite und einer elektrischen Verbindung zwischen der distalen Seite und der Proximalen Seite, wobei die elektrische Verbindung aus einer Vielzahl von einzelnen Drahtleitern besteht und wobei der wenigstens einer optischen Sensor ohne Zwischenverbindung einer gedruckten, fotolithografisch strukturierten oder gepressten elektrischen Leiterplatte direkt auf die elektrische Verbindungsleitung zwischen der distalen und der proximalen Seite aufgebaut ist.

Sie betrifft weiter ein Verfahren zum Herstellen eines Endoskops mit einer Optischen Sensoranordnung mit elektrischen Leiter wobei in einer ersten Phase die elektrischen Leiter vorbereitet werden und in einer zweiten Phase der Bildsensor oder das Kameramodulauf die vorbereiteten Leiter aufgebracht wird.

### Stand der Technik

Für medizinische Untersuchungen, Eingriffe und Analysen werden oft Endoskope eingesetzt. Um mit möglichst kleinen Eingriffen für den Patienten das Endoskop zur Untersuchungs- oder Operationsstelle zu bringen, wird versucht ein Endoskop mit möglichst kleinem Durchmesser einzusetzen. Andererseits besteht die Bestrebung die Ortsauflösung der endoskopischen Kamera zu erhöhen. Weiter besteht die Bestrebung zunehmend Einweg Endoskope herzustellen, welche die Risiken und Kosten einer Sterilisierung ausschliessen. Um Einweg Endoskope zu ermöglichen ist es allerdings notwendig den Aufbau einfach und kostengünstig auszugestalten.

Durch die Fortschritte in der Integration von miniaturisierten CMOS Bildsensoren werden zunehmend Endoskope gebaut welche den CMOS Bildsensor bereits in der Endoskopspitze integrieren. Siehe insbesondere den Artickel "Miniature Form Factor digital-image sensor for endoscopic applications" Von M. Wäny et. Al. Veröffentlicht in der Zeitschrift: SPIE Photonics West in Januar. 2009 unter der Referenz EI09-EI114-9_7249-32. Um die Baugrössen der Bildsensoren zu reduzieren und um einen kompakten Aufbau der Sensoren und Optik in der Endoskopspitze zu ermöglichen können sogenannte "Chip Scale Packaging" Technologien, insbesondere "Through Silicon Via Package" Technologien eingesetzt werden. Diese Technologien ermöglichen es, die Abmasse des Bildsensor Aufbaus auf die Grösse des Sensorchips zu beschränken und Produktionskosten, insbesondere bei kleinen Sensoren zu reduzieren. Diese Aufbautechnologien weisen aber üblicherweise eine zweidimensionale Matrix von Anschlusspunkten , die sogenannten "Ball Grid Array" auf, der zum elektrischen Anschluss auf eine Leiterplatte aufgebracht werden müssen, welche wiederum mit den elektrischen Leitern verbunden werden. Diese Art des Aufbaus verlangt jedoch einen relativ grossen Bauraum und führt zu relativ hohen kosten, so dass sich die Kommerzielle Verbreitung dieser Art Bildsensoren für Endoskopen trotzdem nicht wie gewünscht entwickelt hat.

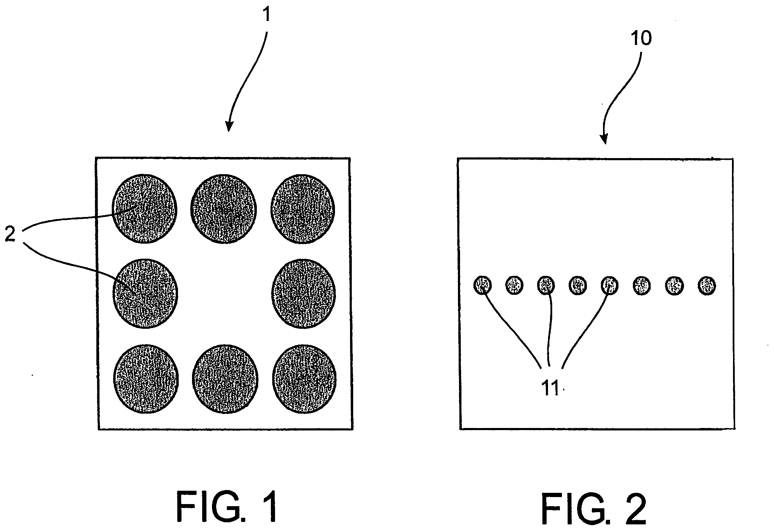

Die amerikanische Patentanmeldung US 2008/0091064 A1 betrifft eine miniaturisierte Endoskopanordnung welche billig hergestellt und mit niedrigem Energieverbrauch betätigt kann werden. Jedoch diese Anordnung benutzt nicht die Herstellungstechnologie der Erfindung, die verschiedene Teilmodule sind durch Kabel verbunden. Die **Abbildung 2****,** beschreibt, dass der Bildsensor ein BGA Array (nach stand der Technik ein zweidimensionales Array) aufweist, sowie auf eine flexible leiterplatte aufgebaut wird. Weiter ist der Bildsensor als "micro ball gridarray" also als 2-dimensionales array beschrieben, und auf einer "Flexiblen Leiterplatte" aufgebracht, nicht direkt auf eine Draht Leitung.

**Die** Bildaufnehmer ist explizit als Standard Bildaufnehmer wie in der mobil Telefon Technik verwendet, wo standardmässig 2 dimensionale BGA Kontacktarrays verwendet werden.

**Der Anspruch** beschreibt ausserdem explizit dass eine Anordnung mit einem Bildsensor der Mittels 2-dimensionaler Kontakt Matrix "micro ball gridarray" elektrisch angeschlossen wird gemeint ist.

Die amerikanische Patentanmeldung US 2005/0285973 A1 beschreibt eine Bildkamera die auf einem flexiblen Schaltung montiert ist. Der Sensor ist mechanisch direkt auf ein FPS montiert, aber nicht elektrisch direkt damit verbunden ist. (Wirebonding notwendig). Ein direktes Aufbringen von Sensor BUMP's auf eine FPC (nicht aber eindimensionales Array, oder eine Draht Leitung). Die **Abbildung 2****,** beschreibt, wie der Sensor zwar mechanisch direkt auf einen FPC aufgebracht wird, die elektrische Verbindung aber mittels Draht Bonden zu erstellen ist. **Es wird** explizit beschrieben dass der Sensor zwar direkt, aber elektrisch NICHT leitend auf eine FPC aufgebracht wird sowie die Reduktion von teilen, allerdings bleibt beschränkt auf eine FPC als Substrat und einen Prozess zum Aufbau auf ein FPC (nicht aber die Details des direkten elektrischen Verbindungsvorganges)

Die europpäische Patentanmeldung EP 1 104 182 A1 betrifft ein Bildempfänger der auf einer wenigstens teilweise flexiblen gedruckte Schaltung aufgebaut ist für eine Endoscopanordnung. Die **Abbildung 3** zeigt den Querschnitt eines Standard BGA type Chip ScalePakcages, also eines Packages mit 2-dymensionaler Kontakt Anordnung, wie dies in den folgenden Figuren 4 und 5 ersichtlich ist.

Weiter wird in dieser Erfindung der Aufbau direkt auf eine FPC beschreiben, nicht aber auf eine parallel Draht Leitung. Ebenso wenig wird eine eindimensionale Kontaktanordnung beschreiben.

Ausgehend von diesem Stand der Technik liegt der Erfindung die Aufgabe zugrunde einen optischen Sensor, insbesondere einen Bildsensor herszustellen, dadurch gekennzeichnet dass, die elektrische Verbindung aus einer Vielzahl von einzelnen Leitern besteht welche ein eindimensionales Netz bilden, und dass, der optischen Sensoranordnung ohne Zwischenverbindung einer elektrischen Leiterplatte direkt auf die besagten elektrischen Leitern mit einer Isolierung aufgebracht wird, wobei die Periode des Netzes der Periode welche in dem Kontaktbereich der elektrischen Leiter aufgefunden wird entspricht.

### Zusammenfassung der Erfindung

Die Erfindung zeigt Mittel und Prozesstechniken, mit denen ein optischer Sensor, vorteilhaft ein Bildsensor oder einen Miniatur Kameramodul bestehend aus einem Bildsensor und einer optischen Linse, direkt auf einen elektrischen Leiter aufgebracht werden kann, sowie ein Verfahren um kostengünstig elektrische Leiter so vorzubereiten dass der optische Sensor durch die beschriebenen Aufbauprozesse auf dieselben montiert werden können.

Deswegen ist der Endoskop mit einem optischen Sensor gemäss der Erfindung dadurch gekennzeichnet dass, dadurch gekennzeichnet dass, die elektrischen Kontakte auf dem optischen Sensor so ausgestaltet sind, dass diese in nur einer Reihe angeordnet sind und dass die Kontakte (11,21) in dieser Reihe mit einer Periode angeordnet sind, welche der Periode der einzelnen Drahtleiter entspricht, die in einem Kontaktbereich der besagten Elektrischen Verbindung aufgefunden wird.

Vorteilhaft sind die besagte Kontakte in der Richtung Orthogonal zur Kontaktreihe länger als in der Richtung der Kontaktreihe.

Vorzugsweise enthält besagter elektrische Leiter ein Flachbandkabel mindestens zwei getrennten elektrischen Leitern.

Das Verfahren gemäss der Erfindung ist dadurch gekennzeichnet dass, während der zweiten Phase thermischaushärtender Kleber mit elektrisch Leitenden Partikeln angebracht ist und welcher während dem aushärten sein Volumen reduziert verwendet wird um die optische Sensoranordnung auf dem Leiter zu befestigen.

Vorzugsweise wird thermisch aushärtender Kleber, welcher mit elektrisch leitenden Partikeln angereichert ist und welcher während dem aushärten sein Volumen reduziert verwendet.

Vorzugsweise wird die elektrische und mechanische Verbindung zwischen den metallen des elektrischen Leiters und der Kontakte des besagten Bildsensors oder Kameramoduls dadurch hergestellt, dass durch mechanischen druck und Anregung mittels Ultraschallenergie eine bleibende Verbindung, besteht

Vorzugsweise werden besagte elektrische Leiter oder besagter Bildsensor oder Kameramodul oder beide Teile, vor dem Verbindungsprozess mittels Plasma aktiviert.

Vorzugsweise wird während der ersten Phase der elektrische Leiter mechanisch festgehalten wird und auf der einen Seite gegen einen mechanischen Anschlag gepresst wird, während von der anderen Seite die Isolierung des Leiters mechanisch abgetragen.

Vorteilhaft wird während der ersten Phase der Abtragung der elektrischen Isolation vom Leiter ebenso ein Teil des elektrischen Leiters abgetragen, so dass eine mindestens teilweise plane Oberfläche auf dem elektrischen Leiter entsteht.

Nach einer Variante dieses Verfahrens, wird während der ersten Phase die elektrische Isolation der Leiter in einem Kontaktbereich mittels Laserbearbeitung entfernt.

Während der zweiten Phase, wird vorzugsweise die elektrische und mechanische Verbindung zwischen den metallen des elektrischen Leiters und der Kontakte der besagten optischen Sensoranordnung dadurch hergestellt, dass durch mechanischen Druck und Anregung mittels Ultraschallenergie eine bleibende Verbindung entsteht.

Während der zweiten Phase kann die elektrische und mechanische Verbindung zwischen den metallen des elektrischen Leiters und der Kontakte der besagten optischen Sensoranordnung dadurch hergestellt werden, dass durch mechanischen Druck und Anregung mittels Ultraschallenergie eine bleibende Verbindung entsteht.

Vorteilhaft werden besagte elektrische Leiter oder besagte optische Sensor oder beide Teile, vor dem Verbindungsprozess mittels Plasma aktiviert.

Während der zweiten Phase kann die elektrische und mechanische Verbindung zwischen dem Anwendung des optischen Sensors und dem Elektrischen Leiter mittels Lötprozess hergestellt werden.

Vorteilhaft wird die Wärmezufuhr zum aufschmelzen des Lotes mittels einer optischen Quelle erzeugt.

Vorzugsweise erfolgt die Wärmezufuhr zum aufschmelzen des Lotes durch Wärmeübertragung einer Heizquelle welche in mechanischem Kontakt zum elektrischen Leiter ist.

### Zusammenfassende Beschreibung der Zeichnungen

Nachstehend ist ein Ausführungsbeispiel der Erfindung anhand der Zeichnung beschrieben. Es zeigt:
Figur 1 zeigt eine Anordnung von elektrischen Anschlüssen in einer zweidimensionalen Anschlussmatrix (Ball Grid Array) gemäss dem Stand der Technik,
Figur 2 zeigt eine Anordnung von elektrischen Anschlüssen in einer eindimensionalen Anschlussmatrix,

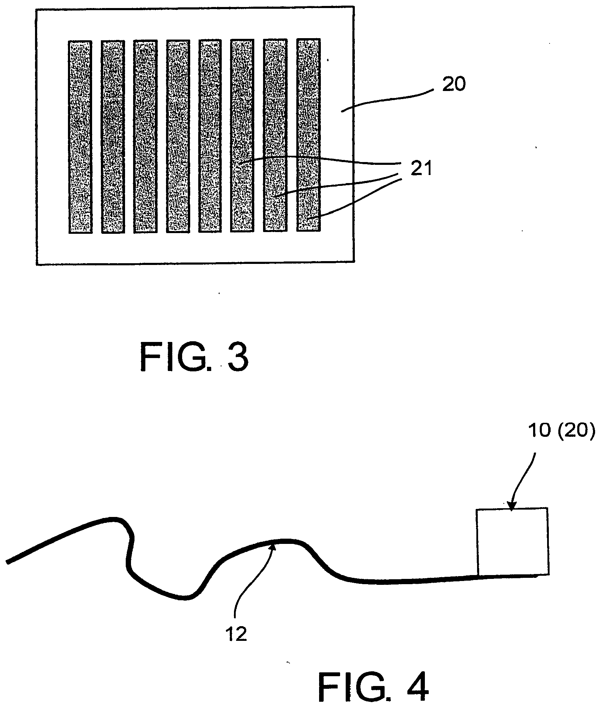

Figur 3 zeigt eine Anordnung von elektrischen Anschlüssen in einer eindimensionalen Anschlussmatrix, und
Figur 4 zeigt eine optische Sensoranordnung, welche direkt auf ein elektrisches Leitungskabel aufgebaut ist.

### Beste Art der Ausführung der Erfindung

Figur 1 zeigt eine Anordnung von elektrischen Anschlüssen 2 auf eine optische Sensoranordnung 1 in einer zweidimensionalen Anschlussmatrix (Ball Grid Array) gemäss dem Stand der Technik.

Figur 2 zeigt die erfindungsgemässe Anordnung von elektrischen Anschlüssen 11 auf eine optische Sensoranordnung 10 in einer eindimensionalen Anschlussmatrix. Die elektrischen Anschlüsse können sowohl in Gold, Kupfer oder aus Zinn oder Zinn Legierungen oder anderen elektrischleitende Materialien ausgebildet werden.

Figur 3 zeigt die erfindungsgemässe Anordnung von elektrischen Anschlüssen 21 auf eine optische Sensoranordnung 20 in einer eindimensionalen Anschlussmatrix in Form einer Kontakzreihe bei welcher die Anschlüsse 21 rechteckig ausgebildet sind so dass die Anschlüsse orthogonal zur Kontaktreihe länger sind als breit. Die elektrischen Anschlüsse können sowohl in Gold, Kupfer oder aus Zinn oder Zinn Legierungen oder anderen elektrischleitenden Materialien ausgebildet werden.

Figur 4 zeigt die erfindungsgemässe Anordnung einer optischen Sensoranordnung 10 (oder 20) welcher direkt auf einem elektrischen Leiter 12 aufgebaut ist.

Die in dieser Beschreibung aufgeführten oder in den Zeichnung dargelegten Realisierungsmöglichkeiten der Erfindung sind einzig als erläuternde Beispiele zu verstehen. Die Beispiele schränken nicht die Generalität der Erfindung ein. Der Schutz der Erfindung ist einzig in den beiliegenden Patentansprüchen dargelegt.

Der Erfindungsgemässe Aufbau von optischen Sensorenanordnungen, Bildsensoren respektive Miniaturkameras genannt auf elektrische Leiter ohne dass eine Elektrische Leiterplatte eingesetzt werden, kann dadurch realisiert werden, dass der Sensor direkt auf eine freigelegte Fläche des elektrischen Leiters, wessen Leiterbahnen im Sensorbereich parallel verlaufen aufgebracht wird. Um dies zu ermöglichen wird im erfindungsgemässen Sensor 10 die Kontaktematrix als eindimensionaler Array, sogenannte Kontaktreihe 11 ausgebildet. Die Kontakte werden in der Richtung orthogonal länger ausgebildet als in der Richtung der Kontaktreihe 21. Optional werden die Kontakte so ausgestaltet dass die Oberfläche der Kontaktpunkte über die Ebene der Sensoranordnungsrückfläche hinausseht, und so dass diese in mindestens einem kleinen Bereich plan oder annähernd plan ausgestaltet ist.

Der elektrische Leiter besteht aus einer Vielzahl von einzelnen Leitern, die entweder über die gestammte Leiterlänge parallel verlaufen (z.B. Flachbandkabel) oder aus elektrischen Leitern die im Bereich in welchem der Sensor aufgebaut wird parallel gelegt werden. Die elektrischen Leiter können sowohl durch photolithografische Prozesse wie auch durch "Extruktions" Prozesse hergestellt werden. Im Falle von durch Extruktion hergestellten elektrischen Leitern, sind diese vor dem Aufbau im Bereich in welchem die Sensoranordnung aufgebracht werden von der Isolation zu befreien. Dies geschieht im erfindungsgemässen Verfahren so, dass der elektrische Leiter mechanisch festgehalten wird und auf der einen Seite gegen eine feste Auflage gepresst wird. Von der freien Seite wird die elektrische Isolation mechanisch, mit Vorteil durch ein rotierendes abrasives Werkzeug, entfernt. Mit der Entfernung der Isolation wird ebenfalls der Leiter soweit abgetragen dass wenigstens ein Teil der runden Leiteroberfläche abgetragen wird und eine wenigstens in einem Teil plane Kontaktoberfläche entsteht. In einem alternativen Verfahren zur vorbereitung der Leiter wird die Isolation mittels Laserbarabeitung abgetragen. In diesem Fall wird mit vorteil lediglich die Isolation abgetragen, nicht aber der leiter, so dass dessen ursprüngliche Form erhalten bleibt. Die Vorbereitung der elektrischen Leiter zur Bestückung der Kameras kann optional so ausgestaltet werden, dass in der gleichen halte Vorrichtung eine Pluralität von elektrischen Leitern fixiert wird die im Verbund zusammengehalten werden um später die Bestückung der optischen Sensorenanordnung parallel ausführen zu können.

Die optische Sensoranordnung, insbesondere einen Bildsensor oder ein Miniatur Kameramodul wird direkt auf den elektrischen Leiter montiert. Im erfindungsgemässen Prozess geschieht dies dadurch dass ein thermisch härtender Kleber, welcher mit elektrisch leitenden Partikeln angereichert ist und welcher beim aushärten an Volumen verliert zwischen den Leiter und der optischen Sensoranordnung aufgetragen wird. Die optische Sensoranordnung wird mit mechanischem Druck gegen den Leiter gepresst, während thermische Energie den Kleber aushärtet. Die Anzahl und Grösse der elektrisch leitenden Partikel ist so zu wählen, dass mit dem bei der Aushärtung applizierten mechanischen Druck Partikel zwischen den jeweiligen elektrischen Leitern und dem entsprechenden Kontakt auf die optische Sensoranordnung Seite eingeklemmt werden und einen OhmischenKontakt zwischen die optische Sensoranordnung und dem entsprechenden Leiter herstellen aber keinen elektrischen Kontakt zu den benachbarten elektrischen Leitern.

In einer alternativen Realisierung des Erfindungsgemässen Aufbauprozesses wird der Sensor ohne Fügemittel direkt auf den elektrischen Leiter aufgebracht. Eine bleibende elektrische und mechanische Verbindung wird dadurch realisiert, dass mittels mechanischem Druck und Ultraschallenergie eine Metallische Verbindung zwischen den Kontakten der optischen Sensoranordnung und den elektrischen Leitern entsteht. Zur besseren Qualität der Verbindung können optional der optischen Sensoranordnung und oder elektrischen Leiter mittels Plasmaprozess gereinigt und aktiviert werden.

In einer weiteren alternativen Realisierung des Erfindungsgemässen Aufbauprozesses wird der Sensor mittels Lötforgang direkt auf den elektrischen Leiter aufgebracht.

Die Anwendung des erfindungsgemäss aufgebauten Bildsensors direkt auf ein elektrisches Kabel ist insbesondere interessant für Anwendungen in medizinischen und industriellen Endoskopanordungen, da die Dimensionen der optischen Sensoranordnung minimal ausfallen, was den Bau von Endoskopen mit kleinem Durchmesser ermöglicht. Ausserdem ermöglicht der Aufbau der Sensoren respektive Kameramodulen direkt auf ein Kabel, ohne eine elektrische Leiterplatte, die Reduktion von Kosten, was die Herstellung von Einweggeräten ermöglicht.

## Patentansprüche

1. Endoskop mit wenigstens einer Optischen Sensoranordnung mit einer distalen Seite und einer proximalen Seite und einer elektrischen Verbindung zwischen der distalen Seite und der proximalen Seite, wobei die elektrische Verbindung aus einer Vielzahl von einzelnen Drahtleitern besteht und wobei der wenigstens einer optischen Sensor ohne Zwischenverbindung direkt auf die elektrische Verbindungsleitung zwischen der distalen und der proximalen Seite aufgebaut ist, **dadurch gekennzeichnet dass**, die elektrischen Kontakte auf dem optischen Sensor so ausgestaltet sind, dass diese in nur einer Reihe angeordnet sind und dass die Kontakte (11,21) in dieser Reihe mit einer Periode angeordnet sind, welche der Periode der einzelnen Drahtleiter entspricht, die in einem Kontaktbereich der besagten Elektrischen Verbindung aufgefunden wird.

2. Endoskop nach Anspruch 1, **dadurch gekennzeichnet dass**, besagte Kontakte (21) in der Richtung Orthogonal zur Kontaktreihe länger sind als in der Richtung der Kontaktreihe.

3. Optische Sensoranordnung nach Anspruch 1, **dadurch gekennzeichnet dass**, besagter elektrische Leiter aus einem Flachbandkabel mit mindestens zwei getrennten elektrischen Leitern besteht.

4. Verfahren zum Herstellen eines Endoskops mit wenigstens einer Optischen Sensoranordnung mit elektrischem Leiter nach einem der Ansprüche 1 bis 3, wobei, in einer ersten Phase die elektrischen Leiter vorbereitet werden und in einer zweiten Phase der Bildsensor oder das Kameramodul auf die vorbereiteten Leiter aufgebracht wird, **dadurch gekennzeichnet dass** während der zweiten Phase thermisch aushärtender Kleber, welcher mit elektrisch leitenden Partikeln angereichert ist und welcher während dem aushärten sein Volumen reduziert verwendet wird um die optische Sensoranordnung auf dem Leiter zu befestigen.

5. Verfahren zum Herstellen eines Endoskops nach Anspruch 4 **dadurch gekennzeichnet dass** während der zweiten Phase, die elektrische und mechanische Verbindung zwischen den metallen des elektrischen Leiters und der Kontakte die besagte optische Sensoranordnung dadurch hergestellt wird, dass durch mechanischen Druck und Anregung mittels Ultraschallenergie eine bleibende Verbindung entsteht.

6. Verfahren zum Herstellen eines Endoskops nach Anspruch 5 **dadurch gekennzeichnet dass** besagter elektrische Leiter und oder besagter Bildsensor oder Kameramodul oder beide Teile, vor dem Verbindungsprozess mittels Plasma aktiviert werden.

7. Verfahren zum Herstellen eines Endoskops nach Anspruch 5 **dadurch gekennzeichnet dass** während der zweiten Phase, die elektrische und mechanische Verbindung zwischen dem der optischen Sensoranordnung und dem Elektrischen Leiter mittels Lötprozess hergestellt wird.

8. Verfahren zum Herstellen eines Endoskops nach Anspruch 7 **dadurch gekennzeichnet dass** die Wärmezufuhr zum aufschmelzen des Lotes mittels einer optischen Quelle erzeugt wird.

9. Verfahren zum Herstellen eines Endoskops nach Anspruch 7 **dadurch gekennzeichnet dass** die Wärmezufuhr zum aufschmelzen des Lotes durch Wärmeübertragung einer Heizquelle welche in mechanischem Kontakt zum elektrischen Leiter ist erfolgt.

10. Verfahren zum Herstellen eines Endoskops nach Anspruch 7 **dadurch gekennzeichnet dass** die Wärmezufuhr zum aufschmelzen des Lotes durch einen Strom von heissem Gas, insbesondere von heisser Luft erfolgt.

## Claims

1. An endoscope comprising at least one optical sensor arrangement having a distal side and a proximal side and an electrical connection between the distal side and the proximal side, wherein the electrical connection consists of a plurality of individual wire conductors and wherein the at least one optical sensor is built up directly on the electrical connection line between the distal and the proximal side without any intermediate connection, **characterized in that** the electrical contacts on the optical sensor are arranged so as to be arranged in only one row and **in that** the contacts (11, 21) in this row are arranged with a period corresponding to the period of the individual wire conductors found in a contact area of said electrical connection.

2. The endoscope according to claim 1, **characterized in that** said contacts (21) are longer in the direction orthogonal to the contact row than in the direction of the contact row.

3. An optical sensor arrangement according to claim 1, **characterized in that** said electrical conductor consists of a ribbon cable having at least two separate electrical conductors.

4. A method of manufacturing an endoscope comprising at least one optical sensor arrangement having an electrical conductor according to any of claims 1 to 3, wherein in a first phase the electrical conductors are prepared and in a second phase the image sensor or the camera module is applied to the prepared conductors, **characterized in that** during the second phase a thermosetting adhesive, which is enriched with electrically conductive particles and reduces its volume during the curing process, is used to fasten the optical sensor arrangement to the conductor.

5. The method of manufacturing an endoscope according to claim 4, **characterized in that** during the second phase the electrical and mechanical connection between the metals of the electrical conductor and the contacts said optical sensor arrangement is manufactured by forming a permanent connection by mechanical pressure and excitation by means of ultrasonic energy.

6. The method of manufacturing an endoscope according to claim 5, **characterized in that** said electrical conductor and/or said image sensor or camera module or both parts are activated through plasma before the connecting process.

7. The method of manufacturing an endoscope according to claim 5, **characterized in that** during the second phase the electrical and mechanical connection between the optical sensor arrangement and the electrical conductor is made by a soldering process.

8. The method of manufacturing an endoscope according to claim 7, **characterized in that** the heat supply for melting the solder is generated by means of an optical source.

9. The method of manufacturing an endoscope according to claim 7, **characterized in that** the heat supply for melting the solder is effected by heat transfer from a heat source which is in mechanical contact with the electrical conductor.

10. The method of manufacturing an endoscope according to claim 7, **characterized in that** the heat supply for melting the solder is effected by a stream of hot gas, in particular hot air.

## Revendications

1. Endoscope comprenant au moins un agencement de capteur optique avec un côté distal et un côté proximal et une liaison électrique entre le côté distal et le côté proximal, sachant que la liaison électrique est composée d'une pluralité de fils conducteurs individuels et sachant que l'au moins un capteur optique est monté directement sur la ligne de liaison électrique entre le côté distal et le côté proximal sans liaison intermédiaire, **caractérisé en ce que** les contacts électriques sur le capteur optique sont configurés de telle sorte que ceux-ci ne soient disposés qu'en une rangée et **en ce que** les contacts (11, 21) dans cette rangée soient disposés selon une période qui correspond à la période des fils conducteurs individuels qui se trouve dans une zone de contact de ladite liaison électrique.

2. Endoscope selon la revendication 1, **caractérisé en ce que** lesdits contacts (21) sont plus longs dans la direction orthogonale à la rangée de contacts que dans la direction de la rangée de contacts.

3. Agencement de capteur optique selon la revendication 1, **caractérisé en ce que** ledit conducteur électrique est composé d'un câble plat comportant au moins deux conducteurs électriques séparés.

4. Procédé de fabrication d'un endoscope comprenant au moins un agencement de capteur optique à conducteur électrique selon l'une des revendications 1 à 3, sachant que, dans une première phase, les conducteurs électriques sont préparés et, dans une deuxième phase, le capteur d'image ou le module de caméra est apposé sur les conducteurs préparés, **caractérisé en ce que**, pendant la deuxième phase, une colle thermodurcissable qui est enrichie de particules électriquement conductrices et qui diminue de volume pendant le durcissement est utilisée pour fixer l'agencement de capteur sur le conducteur.

5. Procédé de fabrication d'un endoscope selon la revendication 4, **caractérisé en ce que**, pendant la deuxième phase, la liaison électrique et mécanique entre les métaux du conducteur électrique et les contacts dudit agencement de capteur optique est réalisée **en ce qu'**une liaison permanente est créée par pression mécanique et sollicitation au moyen d'énergie à ultrasons.

6. Procédé de fabrication d'un endoscope selon la revendication 5, **caractérisé en ce que** ledit conducteur électrique et/ou ledit capteur d'image et module de caméra ou les deux parties sont activés par plasma avant le processus de liaison.

7. Procédé de fabrication d'un endoscope selon la revendication 5, **caractérisé en ce que**, pendant la deuxième phase, la liaison électrique et mécanique entre l'agencement de capteur optique et le conducteur électrique est réalisée par brasage.

8. Procédé de fabrication d'un endoscope selon la revendication 7, **caractérisé en ce que** l'apport de chaleur pour la fusion de la brasure est effectué au moyen d'une source optique.

9. Procédé de fabrication d'un endoscope selon la revendication 7, **caractérisé en ce que** l'apport de chaleur pour la fusion de la brasure est effectué par transfert de chaleur d'une source de chaleur qui est en contact mécanique avec le conducteur électrique.

10. Procédé de fabrication d'un endoscope selon la revendication 7, **caractérisé en ce que** l'apport de chaleur pour la fusion de la brasure est effectué par un flux de gaz chaud, en particulier d'air chaud.
